# FASCICULE DE BREVET EUROPEEN

(11) **EP 0 967 290 B1**
(45) Date de publication et mention de la délivrance du brevet: **30.07.2003**
(21) Numéro de dépôt: 99401542.8
(22) Date de dépôt: 22.06.1999
(51) Int. Cl.: C12P 7/18

(54) **Procédé de production d'Erythritol par fermentation discontinue alimentée répétée**
Verfahren zur Herstellung von Erythritol mittels wiederholten fedbatch Fermentation
Process for producing erythritol by repeated fedbatch fermentation

(30) Priorité: 24.06.1998 FR 9807998
(43) Date de publication de la demande: 29.12.1999
(73) Titulaire: Roquette Frères, 62136 Lestrem (FR)
(72) Inventeur: Seigueilha, Laurent, 163, rue de Lille, 59130 Lambersart (FR)
(74) Mandataire: Boulinguiez, Didier

(56) Documents cités:
- EP-A- 0 136 802
- EP-A- 0 136 803
- EP-A- 0 136 804
- EP-A- 0 327 342

## Description

La présente invention concerne un procédé de production d'érythritol par fermentation discontinue alimentée et répétée de sucres par des micro-organismes producteurs d'érythritol.

On entend par fermentation discontinue alimentée (ou fed-batch), une fermentation dans laquelle on alimente des micro-organismes par ajouts successifs de substrats et dans laquelle le produit et les co-produits de la fermentation restent dans le milieu, jusqu'en fin de fermentation.

On entend par fermentation discontinue alimentée répétée, une fermentation discontinue alimentée dans laquelle on retire la fraction du milieu de fermentation renfermant les micro-organismes, pour l'utiliser comme inoculum pour une fermentation discontinue alimentée suivante.

Le terme substrats se définit comme étant l'ensemble des éléments nutritifs que l'on introduit dans les milieux de fermentation. Au sens de l'invention, les substrats sont principalement les sources carbonées et azotées directement assimilables par les micro-organismes producteurs d'érythritol.

La préparation industrielle de l'érythritol repose principalement sur des procédés fermentaires qui mettent en oeuvre des levures ou des champignons unicellulaires, et ce, à partir d'hydrocarbures ou de sucres comme sources de carbone directement assimilables.

Par sucres, on entend dans la présente invention, toutes les sources carbonées directement assimilables par les micro-organismes producteurs d'érythritol. De tels sucres sont par exemple choisis dans le groupe constitué par le glucose, le saccharose, le fructose, le maltose, le xylulose et le maltulose, seuls ou en mélange. Par extension, on entend également par sucres certains sucres-alcools (ou polyols) tels le mannitol ou le sorbitol qui, étant assimilées par lesdits micro-organismes, conduiront également à la production d'érythritol.

Les micro-organismes producteurs d'érythritol à partir de sucres, sont principalement des levures capables de supporter des pressions osmotiques importantes et appartenant aux genres *Moniliella, Aureobasidium, Torulopsis, Candida, Trigonopsis, Trichosporon, Yallowia*... sans que cette liste ne soit limitative. Ces levures sont - choisies préférentiellement dans la famille des micro-organismes dits osmotolérants, et sont par exemple isolées du miel.

De manière générale, les procédés microbiologiques de production d'érythritol peuvent mettre en oeuvre deux modes de fermentation différents : le mode discontinu et le mode continu.

Dans les modes discontinus (ou batch), tous les substrats nécessaires à l'alimentation des micro-organismes sont introduits au début de la fermentation, et le produit et les co-produits de la fermentation sont extraits en fin de fermentation.

De tels modes de fermentation discontinus ont été mis en oeuvre pour la production d'érythritol, et sont par exemple décrits dans les brevets EP 136 803 et JP 61-31 091.

Cependant, les inconvénients des modes discontinus pour la production d'érythritol que l'on relève dans ces deux brevets, sont d'une part la longue durée de la fermentation, de l'ordre de une à deux semaines, et d'autre part la nécessité de gérer de grands volumes de milieux de fermentation, pour une productivité qui ne dépasse pas les 1,5 grammes par litre et par heure (g/l/heure) et un rendement de l'ordre de 10 à 30 %, d'où des coûts de production élevés.

Dans les modes continus, tous les substrats de fermentation sont ajoutés de manière continue dans le réacteur, et des fractions du milieu de fermentation sont extraites avec le même débit que l'apport en substrats, de manière à travailler à volume constant.

Un mode continu a été mis en oeuvre pour la production d'érythritol, et est décrit dans le brevet US 4 923 812. Ce procédé présente l'avantage de résoudre la majorité des inconvénients du mode discontinu, et ainsi permet un gain non négligeable de productivité et de rendement.

Cependant, ces modes continus ne permettent pas dobtenir des produits extrêmement purs, puisque l'érythritol soutiré est obligatoirement contaminé par les substrats réintroduits en cours de fermentation.

Ainsi, pour la fermentation du glucose par une souche d'*Aureobasidium*, les valeurs présentées dans les exemples de ces deux brevets illustrent ce gain de rendement et de productivité, puisque les rendements en érythritol sont compris entre 45 et 50 % et les productivités sont de l'ordre de 4 à 4,5 g/l/heure. Mais on retrouve également avec l'érythritol produit, environ 10 % de glucose résiduel non consommé, et en outre, les fortes pressions osmotiques subies par le micro-organisme ont nécessairement pour conséquence d'induire la synthèse de glycérol, de ribitol et d'autres polyols avec celle de l'érythritol.

Par conséquent, l'inconvénient majeur des procédés de fermentation continus est la nécessité de purifier l'érythritol. En outre, les techniques de purification ne permettent que difficilement de séparer l'érythritol des coproduits de la fermentation, et pas davantage des sucres résiduels non assimilés par les micro-organismes.

De ce fait, on trouve toujours associé, à ces procédés de fermentation continus, des installations de purification complexes, lourdes et coûteuses.

Par ailleurs, tous les spécialistes s'accordent à reconnaître que les procédés fermentaires de production d'érythritol soulèvent certaines difficultés, que ni le mode discontinu, ni le mode continu n'ont totalement résolues.

Par exemple, pour diminuer les coûts de mise en oeuvre, tous les procédés de fermentation sont basés sur l'utilisation de milieux de fermentation dont la teneur en sucres est maximale

En effet, les micro-organismes producteurs d'érythritol peuvent supporter les hautes teneurs en sucres des substrats de fermentation. Les fortes pressions osmotiques qui en résultent alors conduisent lesdits micro-organismes à synthétiser des polyols dont l'érythritol, mais aussi le glycérol, l'arabitol et/ou le ribitol.

Le problème est qu'à trop forte dose en sucres assimilables, la saturation des voies métaboliques induit l'instabilité des cultures microbiennes et une plus grande mortalité des micro-organismes. De plus, dans ces conditions opératoires, tous les micro-organismes producteurs d'érythritol produisent également d'autres polyols en proportion élevée (jusqu'à 20 % ou plus en ribitol et/ou en glycérol). Dans certains cas, quand la déviation des voies métaboliques est trop importante, l'érythritol devient lui-même un co-produit de la fermentation.

Tous les efforts des spécialistes en fermentation portent donc sur la recherche des conditions opératoires qui conduisent à concilier les meilleurs rendement et productivité en érythritol des micro-organismes, avec leur niveau de tolérance aux concentrations maximales en sucres.

A côté des procédés conventionnels de fermentation discontinus ou continus, il existe un troisième mode de fermentation, appelé mode de fermentation discontinu alimenté, que l'on choisi spécifiquement lorsque l'on constate que la variation de la concentration en l'un des substrats de la fermentation en affecte le rendement ou la productivité, ce qui est le cas du glucose pour la production d'érythritol par voie fermentaire. Ce système discontinu alimenté permet donc de résoudre les problèmes liés à l'inhibition par l'un des substrats de la fermentation.

Ce procédé consiste à introduire les sucres graduellement dans le milieu de fermentation des micro-organismes, en ne réalisant aucun soutirage, et en laissant ainsi le produit dans le fermenteur, en l'occurrence ici l'érythritol, jusqu'à la fin de la fermentation.

Les brevets EP 136 802 et EP 136 804 décrivent un procédé de production d'un mélange de polyols (érythritol, glycérol, ribitol) par la fermentation d'un sucre, préférentiellement le glucose, le saccharose, le fructose ou le maltose, par *Moniliella tomentosa var pollinis*, en utilisant le système discontinu alimenté et répété. La préculture servant à ensemencer le fermenteur est éventuellement prélevée lors d'une fermentation précédenté.

Cependant, le brevet EP 136 802 donne les conditions de fermentation qui favorisent la production d'érythritol, de glycérol et de ribitol à partir de concentrations en sucres, préférentiellement le glucose, supérieures à 30 % en poids/volume, et les conditions qui favorisent surtout la production de ribitol par le contrôle du degré d'aération du milieu de fermentation.

Le brevet EP 136 804 décrit, pour la production de ce même mélange de polyols, l'ajout de 40 % à 80 % en poids/volume de sucres au milieu de fermentation, la concentration en sucres au départ étant fixée entre 20 et 35 % en poids/volume, avec ajout progressif de manière à ce que la quantité totale de sucres additionnés soit au moins de 40 % en poids/volume.

Cependant, les problèmes majeurs rencontrés dans la mise en oeuvre de ces procédés sont le retard de croissance du micro-organisme, la durée de la fermentation qui peut atteindre jusqu'à douze jours, temps nécessaire à la consommation totale du substrat, et la présence de co-produits, qui pénalise encore les rendements en érythritol et nécessite de mettre en oeuvre une purification particulière.

En effet, ces procédés conduisent surtout à un mélange d'érythritol, de glycérol et de ribitol. D'-autre part, la mise en oeuvre de concentrations aussi élevées en sucres (supérieures à 40 % poids/volume) ne permet pas d'obtenir facilement et majoritairement l'érythritol.

La présente invention a donc pour but de résoudre ces problèmes et de fournir un procédé de production d'érythritol qui réponde mieux que ceux qui existent déjà, aux diverses contraintes de la pratique.

Le procédé de production d'érythritol mis au point par la société Demanderesse permet d'assurer la production quasi-exclusive d'érythritol, et est basé sur une succession de fermentations avec apport intermittent de substrats et avec recyclage de la biomasse, en maintenant une concentration en sucres totaux à une valeur inférieure à 200 g/l.

On entend par concentration en sucres totaux, la somme des concentrations en sucres résiduels, non consommés par les micro-organismes, et des concentrations en sucres amenés par l'ajout de substrats.

L'invention porte plus particulièrement sur un procédé de production d'érythritol par fermentation discontinue alimentée et répétée de sucres par des micro-organismes producteurs d'érythritol, caractérisé par le fait qu'il comprend les étapes suivantes :
a. Démarrage de la fermentation en introduisant lesdits micro-organismes dans un milieu de fermentation renfermant une concentration en sucres inférieure à 200 g/l,
b. Poursuite de la fermentation en conduisant au moins un cycle de fermentation qui comprend :
   - au moins une phase d'ajout des substrats de manière à maintenir la concentration en sucres totaux à une valeur inférieure à 200 g/l,
   - au moins une phase de séparation du milieu de fermentation en une fraction concentrée en micro-organismes et une autre fraction enrichie en érythritol, après consommation totale des sucres,
   - au moins une phase de recyclage des micro-organismes récupérés et concentrés dans le milieu de fermentation,
c. Collecte de l'ensemble des fractions enrichies en érythritol ainsi obtenues.

La première étape a. du procédé conforme à l'invention consiste à démarrer la production d'érythritol par fermentation discontinue alimentée répétée de sucres par des micro-organismes producteurs d'érythritol en introduisant lesdits micro-organismes dans un milieu de fermentation renfermant une concentration en sucres inférieure à 200 g/l.

Ce faisant, la société Demanderesse a vaincu un préjugé technique en choisissant de mettre en oeuvre des conditions de fermentation contraires à celles que les spécialistes recommandent d'utiliser, c'est-à-dire avec une concentration en sucres nettement inférieure aux conditions de fermentation habituellement définies et optimisées pour la production d'érythritol par ces micro-organismes osmotolérants.

Après de longues recherches, la société Demanderesse a en effet mis en évidence que pour la production d'érythritol par voie fermentaire, et afin d'éviter de saturer les voies métaboliques, de produire des inhibiteurs de croissance et surtout pour limiter la co-production d'autres polyols, il fallait démarrer la fermentation avec des concentrations en sucres inférieures à 200 g/l.

La concentration minimum en sucres nécessaire à un démarrage de la fermentation dans des conditions acceptables est déterminée par le micro-organisme producteur d'érythritol choisi.

Pour certains micro-organismes producteurs d'érythritol comme les levures du genre *Moniliella*, par exemple, on choisira préférentiellement des valeurs comprises entre 100 g/l et 150 g/l.

A côté de ces conditions de démarrage de la fermentation pour la production d'érythritol, une des caractéristiques importantes de l'invention est le contrôle des ajouts en substrats dans les milieux de production, afin de maintenir la concentration en sucres totaux à une valeur qui ne dépasse pas 200 g/l, ceci afin de continuer à assurer une production préférentielle d'érythritol.

La seconde étape *b*. du procédé conforme à l'invention consiste alors en la poursuite de la fermentation en conduisant au moins un cycle de fermentation qui comprend :
- au moins une phase d'ajout des substrats de manière à maintenir la concentration en sucres totaux à une valeur inférieure à 200 g/l,
- au moins une phase de séparation du milieu de fermentation en une fraction concentrée en micro-organismes et une autre fraction enrichie en érythritol, après consommation totale des sucres,
- au moins une phase de recyclage des micro-organismes récupérés et concentrés dans le milieu de fermentation.

La société Demanderesse a donc eu le mérite de montrer que l'on maintient la production quasi-exclusive d'érythritol, en conduisant au moins un cycle de fermentation dans -lequel on réalise au moins une étape consistant à ajouter des substrats de manière à maintenir la concentration en sucres totaux à une valeur inférieure à 200 g/l.

Cet ajout de substrats est réalisé lorsque la concentration en sucres atteint une valeur minimale, choisie en fonction du micro-organisme considéré.

La société Demanderesse a en effet remarqué que la tolérance des micro-organismes aux basses concentrations en sucres variait d'un micro-organisme à l'autre, car si lesdits micro-organismes supportent de hautes concentrations en sucres, leur croissance diminue et dans certains cas stoppe lorsque la teneur en sucres du milieu de fermentation est faible.

On adapte donc la phase d'ajout de sucres en fonction des capacités fermentaires des micro-organismes considérés, de manière à réintroduire du substrat lorsque la concentration résiduelle en sucres est suffisamment diminuée, voire nulle, sans que la croissance des micro-organismes considérés ne soit retardée, ou stoppée.

Pour certains micro-organismes producteurs d'érythritol comme les levures du genre *Moniliella*, par exemple, on réalisera préférentiellement l'ajout de substrats lorsque la concentration en sucres totaux atteint une valeur comprise entre 0 et 50 g/l.

L'ajout est réalisé au moins une fois, en ramenant à chaque fois un volume supplémentaire de substrats au volume précédent de la fermentation

Conformément au procédé discontinu alimenté répété selon l'invention, ce volume supplémentaire est apporté sans soutirage de l'érythritol, qui continue donc à s'accumuler dans le fermenteur.

Un avantage du procédé conforme à l'invention est la rapidité de consommation du substrat et la conversion élevée des sucres, essentiellement en érythritol, les co-produits n'étant retrouvés qu'à l'état de traces. En effet, le maintien de la concentration en sucres à une valeur qui ne dépasse pas 200 g/l conduit à sa consommation rapide par les micro-organismes, et donc à une productivité et une richesse élevées en érythritol sans synthèse de co-produits.

Le procédé conforme à l'invention permet donc de réduire fortement la durée de chacune des étapes de la fermentation, d'assurer une richesse élevée en érythritol et aussi de contrôler aisément le développement de la biomasse.

Le cycle de fermentation du procédé conforme à l'invention comprend également au moins une phase de séparation du milieu de fermentation en une fraction concentrée en micro-organismes et une autre fraction enrichie en érythritol, après consommation totale des sucres.

Cette phase permet de provoquer surtout la consommation totale en sucres par les micro-organismes producteurs d'érythritol, ce qui arrête la fermentation et permet d'éviter de contaminer l'érythritol collecté par des sucres résiduels.

On réalise la séparation entre les micro-organismes et la fraction enrichie en érythritol par tout moyen connu par ailleurs de l'homme du métier, par exemple par micro-filtration, en utilisant des membranes dont le diamètre des pores est adapté à la taille du micro-organisme considéré (au moins 1 mm) ou par centrifugation dans un intervalle de 1 000 G à 10 000 G.

La solution clarifiée, enrichie en érythritol, obtenue au terme de cette étape de séparation d'avec les micro-organismes, constitue une partie de l'érythritol produit.

Le cycle de fermentation du procédé conforme à l'invention comprend enfin au moins une phase de recyclage des micro-organismes récupérés et concentrés dans le milieu de fermentation.

On réalise le réensemencement du fermenteur avec la biomasse ainsi récupérée et concentrée, afin de réinitialiser un nouveau cycle de fermentation.

L'avantage de cette étape de recyclage de la biomasse est de réintroduire une biomasse dont les capacités de fermentation sont entières, et d'éviter par là même un surcoût, de même qu'un retard de mise en oeuvre qu'occasionnerait la préparation d'une nouvelle culture des micro-organismes producteurs d'érythritol.

Au terme de ces successions d'étapes, et de l'épuisement des capacités fermentaires des micro-organismes, la troisième étape *c*. du procédé conforme à l'invention consiste alors en la collecte de l'ensemble des fractions enrichies en érythritol ainsi obtenues.

Les polysaccharides formés en petite quantité au cours de la fermentation peuvent être éliminées par les techniques classiques d'ultra- ou de nano-filtration. Les faibles quantités de glucose résiduel ou de contaminants de la fermentation tel l'acétoïne sont quant à elles traitées par toute technique connue en soi, par exemple par leur transformation en acides au moyen d'un traitement en conditions alcalines à une température comprise entre 100°C et 130°C, pendant quelques minutes à quelques heures, en fonction de la température choisie. Les acides formés sont alors facilement éliminés lors d'une déminéralisation.

La récupération de la totalité de l'érythritol issu de toutes les fractions clarifiées est effectuée par tout moyen connu en soi par l'homme du métier, par exemple par concentration à 60 % et plus, suivi de la cristallisation de l'érythritol, tel que décrit dans ROXBURG et al, 1956, Canadian J. Tech., 134, 248-253.

### EXEMPLE

On ensemence trois erlens de 500 ml avec une culture, sur boîte de Pétri, de micro-organismes producteurs d'érythritol (en l'occurrence ici une *Monilella tomentosa* souche CBS L61.67 laissée 22 heures à 37°C), le milieu de culture étant composé de 50 g/l de glucose, 5 g/l d'extraits de levures, 5 g/l de liqueur de corn steep, 3 g/l de KH2PO4 et 1 g/l de MgSO4 et complémenté par 10 ml de NH40H à 20 % de manière à amener le pH initial à une valeur de 6.

On place les erlens sur un agitateur orbital à 150 rotations par minutes (rpm) pendant 20 heures à 37°C.

La préculture ainsi obtenue est introduite dans un fermenteur de 20 litres maintenu à 37°C, agité à 750 rpm et aéré à 15 litres/min, contenant 15 1 du milieu de culture dont la composition est identique au milieu décrit ci-avant. La durée de cette sub-culture est de 22 heures.

Le premier cycle de fermentation est mené en régulant constamment la fermentation dans le fermenteur de 20 litres à un pH supérieur à 3,5, à l'aide de KOH 5 N, et l'agitation fixée à 750 rpm et l'aération à 15 litres/min.

Le démarrage dudit premier cycle est effectuée dans un volume effectif de 14,5 litres, contenant 1,5 litres d'inoculum provenant du fermenteur de subculture., 1500 g de glucose, pour l'amener à une concentration de 100 g/l dans le fermenteur, 150 g d'extraits de levures, 7 g de (NH4)2SO4 et 10 ml de KOH 5 N permettant de porter le pH de départ de ce premier cycle de fermentation à 6.

Deux ajouts de solutions contenant 1500 g de glucose chacune sont effectués successivement après 23 heures (le glucose résiduel est à une concentration de 50 g/l) et 47 heures (le glucose résiduel est à une concentration de 27 g/l) de fermentation.

Après 75 heures de fermentation, le volume final est de 18 litres, et le milieu contient 50 g de glucose résiduel et 2 000 g d'Erythritol, soit un rendement pondéral de 44 % et une productivité de 1,5 g/l/h pour ce premier cycle de production.

Les co-produits sont :

| NATURE | CONCENTRATION |
|---|---|
| Polysaccharides | 0,2 % |
| Glycérol | 1 % |
| Ribitol | 1 % |
| Théhalose | 0,3 % |
| Arabitol | - |
| Acide gluconique | - |
| Sorbitol | - |
| Ethanol | - |

Les cinq heures suivantes sont employées pour traiter le moût de fermentation sur un module de micro-filtration tangentielle, fonctionnant avec une membrane à 0,22 mm de seuil de coupure. 10 litres de filtrat sont récupérés et la biomasse ainsi concentrée est recyclée dans le fermenteur.

Un nouveau cycle de production est alors démarré par un premier ajout de 8 litres de solution contenant 2800 g de glucose et 24 g d'extraits de levures à 80 heures de fermentation (temps mesuré par rapport à l'heure de mise en route du procédé fermentaire).

Un second ajout de 1500 g de glucose est effectué à 120 heures de fermentation. Le taux de glucose résiduel est de 50 g/l.

A 150 heures de fermentation, le milieu contient 5 g/l de glucose résiduel et 2620 g d'érythritol, pour un volume de 18,6 litres, soit pour ce second cycle, un rendement de 42 % et une productivité de 1,4 g/l/h.

Les co-produits sont :

| NATURE | CONCENTRATION |
|---|---|
| Polysaccharides | 0,3 % |
| Glycérol | 2,7 % |
| Ribitol | 0,7 % |
| Théhalose | 0,3 % |
| Arabitol | 0,8 % |
| Acide gluconique | - |
| Sorbitol | - |
| Ethanol | - |

L'ensemble du moût est filtré de la même manière qu'au terme du premier cycle, et l'érythritol obtenu rassemblé avec l'érythritol collecté lors du premier cycle.

## Revendications

1. Procédé de production d'érythritol par fermentation discontinue alimentée et répétée de sucres par des micro-organismes producteurs d'érythritol, **caractérisé par le fait qu'**il comprend les étapes suivantes :
a. Démarrage de la fermentation en introduisant lesdits micro-organismes dans un milieu de fermentation renfermant une concentration en sucres inférieure à 200 g/l,
b. Poursuite de la fermentation en conduisant au moins un cycle de fermentation qui comprend :
- au moins une phase d'ajout des substrats de manière à maintenir la concentration en sucres totaux à une valeur inférieure à 200 g/l,
- au moins une phase de séparation du milieu de fermentation en une fraction concentrée en micro-organismes et une autre fraction enrichie en érythritol, après consommation totale des sucres,
- au moins une phase de recyclage des micro-organismes récupérés et concentrés dans le milieu de fermentation,
c. Collecte de l'ensemble des fractions enrichies en érythritol ainsi obtenues.

2. Procédé selon la revendication 1, **caractérisé par le fait que** l'on démarre la fermentation dans l'étape a. en introduisant lesdits micro-organismes dans un milieu de fermentation renfermant une concentration en sucres comprise entre 100 g/l et 150 g/l.

3. Procédé selon l'une ou l'autre des revendications 1 et 2, **caractérisé par le fait que** l'on ajoute les substrats dans l'étape *b*. lorsque la concentration en sucres totaux atteint une valeur comprise entre O et 50 g/l.

## Patentansprüche

1. Verfahren zur Herstellung von Erythritol durch diskontinuierliche zugespeiste und wiederholte Fermentation von Zuckern durch Erytritol erzeugende Mikroorganismen, **gekennzeichnet durch** die folgenden Schritte:
a) Starten der Fermentation **durch** Einführung der Mikroorganismen in ein Fermentationsmedium, das eine Konzentration an Zuckern unter 200 g/l enthält,
b) Weiterführung der Fermentation, indem mindestens ein Fermentationszyklus ausgeführt wird, der folgendes umfaßt:
- mindestens eine Phase des Zusetzens von Substraten so, daß die Konzentration an Gesamtzuckern auf einem Wert unter 200 g/l gehalten wird,
- mindestens eine Phase der Trennung des Fermentationsmediums in eine hinsichtlich Mikroorganismen konzentrierte Fraktion und eine andere Fraktion, die an Erytritol angereichert ist, nach vollständigem Verbrauch der Zucker,
- mindestens eine Phase der Rezyklierung der rückgewonnenen und konzentrierten Mikroorganismen in das Fermentationsmedium,
c) Sammeln aller auf diese Weise erhaltenen an Erythritol angereicherten Fraktionen.

2. Verfahren nach Anspruch 1, **dadurch gekennzeichnet, daß** man die Fermentation im Schritt a) startet, indem man die Mikroorganismen in ein Fermentationsmedium einführt, das eine Konzentration an Zuckern enthält, die zwischen 100 g/l und 150 g/l beträgt.

3. Verfahren nach einem der Ansprüche 1 und 2, **dadurch gekennzeichnet, daß** man die Substrate im Schritt b) zusetzt, wenn die Konzentration an Gesamtzuckern einen Wert zwischen 0 und 50 g/l erreicht.

## Claims

1. Method of producing erythritol by fed-batch and repeated fermentation of sugars by microorganisms which produce erythritol, **characterised by** the fact that it comprises the following stages:
a) starting the fermentation by introducing said microorganisms into a fermentation medium containing a concentration of sugars lower than 200 g/l,
b) continuing the fermentation by carrying out at least one fermentation cycle which comprises:
- at least one phase of adding substrates so as to keep the concentration of total sugars at a value lower than 200 g/l,
- at least one phase of separating the fermentation medium into a concentrated fraction of microorganisms and another fraction enriched with erythritol, after total consumption of the sugars,
- at least one phase of recycling recovered and concentrated microorganisms in the fermentation medium,
c) collecting all the fractions enriched with erythritol thus obtained.

2. Method according to claim 1, **characterised by** the fact that the fermentation is started in stage a) by introducing said microorganisms into a fermentation medium containing a concentration of sugars of between 100 g/l and 150 g/l.

3. Method according to one or other of claims 1 and 2, **characterised by** the fact that the substrates are added in stage b) when the concentration of total sugars reaches a value of between 0 and 50 g/l.
